Europäisches Patentamt

European Patent Office

Office européen des brevets

(11) Publication number: **0 416 615 B1**

# EUROPEAN PATENT SPECIFICATION

(45) Date of publication of patent specification: **06.12.95**  (51) Int. Cl.⁶: **C07C 21/18**, C07C 17/00

(21) Application number: **90117182.7**

(22) Date of filing: **06.09.90**

(54) **Process for preparing chlorotrifluoroethylene.**

(30) Priority: **06.09.89 JP 232374/89**
          **13.03.90 JP 61814/90**

(43) Date of publication of application:
      **13.03.91 Bulletin  91/11**

(45) Publication of the grant of the patent:
      **06.12.95 Bulletin  95/49**

(84) Designated Contracting States:
      **DE FR GB IT**

(56) References cited:
      **GB-A- 1 030 945**
      **US-A- 2 704 775**
      **US-A- 2 864 873**
      **US-A- 3 636 173**

      **CHEMICAL ABSTRACTS, vol. 111, no. 19, 6th
      November 1989, page 675, abstract no.
      173591g, Columbus, Ohio, US; & JP-A-01 29
      328 (ASAHI GLASS CO., LTD) 31-01-1989**

(73) Proprietor: **DAIKIN INDUSTRIES, LIMITED
      Umeda Center Building,
      4-12 Nakazaki-nishi 2-chome,
      Kita-ku
      Osaka-shi,
      Osaka-fu 530 (JP)**

(72) Inventor: **Morikawa, Yutaka
      5-2-7 Minaminaruse
      Machida-shi,
      Tokyo-to (JP)**
      Inventor: **Ikawa, Tsuneo
      728, Nikaido
      Kamakura-shi,
      Kanagawa-ken (JP)**
      Inventor: **Ueda, Wataru
      Sanhowaito M206-21,
      2-24-4, Minaminaruse
      Machida-shi,
      Tokyo-to (JP)**

(74) Representative: **Hansen, Bernd, Dr.
      Dipl.-Chem. et al
      Hoffmann, Eitle & Partner
      Patent- und Rechtsanwälte,
      Postfach 81 04 20
      D-81904 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

**Description**

The present invention relates to a process for preparing chlorotrifluoroethylene. More particularly, the present invention relates to a process for preparing chlorotrifluoroethylene (hereinafter referred to as "CTFE") from 1,1,2-trichloro-1,2,2-trifluoroethane (CFC-113) the use of which is and will be limited.

Description of the Related Art

Various methods are known for preparing CTFE. For example, Japanese Patent Publication Nos. 5207/1982 and 5208/1982 disclose a liquid phase process comprising dechlorinating 1,1,2-trichloro-1,2,2-trifluoroethane with zinc, Japanese Patent Publication No. 2132/1965 discloses co-pyrolysis of dichlorofluoromethane and chlorodifluoromethane, and Japanese Patent Kokai Publication No. 185734/1985 discloses a gas phase process comprising dechlorinating 1,1,2-trichloro-1,2,2-trifluoroethane with hydrogen in the presence of various active carbon as catalysts. In the gas phase dechlorination of 1,1,2-trichloro-1,2,2-trifluoroethane, a catalyst comprising a noble metal such as Pd carried on a support can be used as disclosed in Japanese Patent Publication No. 8454/1968.

By the liquid phase process using zinc, although CTFE can be obtained at a high yield, treatment of by-produced zinc chloride is troublesome. The co-pyrolysis gives CTFE in a low yield. In the gas phase process using active carbon, a space velocity cannot be larger than 500 $hr^{-1}$ so that the productivity is low. In the gas phase dechlorination using Pd, Pd is expensive and deactivated in a short reaction time, and the reaction should be carried out at a contact time of 10 to 60 seconds so that the productivity is low. Further, a yield of CTFE is unsatisfactory.

US-A-2704775 discloses preparing chlorotrifluoroethylene by reacting 1,1,2-trichloro-1,2,2-trifluoroethane with hydrogen in the presence of nickel, cobalt, copper, platinum, palladium or activated carbon as the catalyst, and of these nickel is the preferred catalyst, especially in the form of finely divided metal, as for instance Raney nickel.

US-A-2864873 refers to process for preparing chlorotrifluoroethylene by reacting 1,1,2-trichloro-1,2,2-trifluoroethane with hydrogen in the presence of a catalyst, which comprises a metal of the group consisting of copper and nickel compulsory together with an oxide of chromium.

SUMMARY OF THE INVENTION

One object of the present invention is to provide a novel gas phase process for preparing CTFE in a high yield.

Another object of the present invention is to provide a novel gas phase process for preparing CTFE in which activity of a catalyst is maintained for a long reaction time and CTFE is produced with high productivity.

These and other objects of the present invention are achieved by a process for preparing CTFE according to Claim 1.

Preferred embodiments thereof are subject matter of Claims 2 to 9.

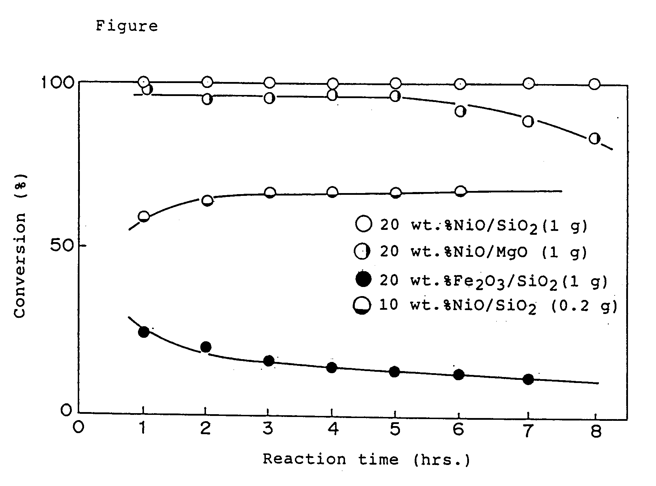

BRIEF DESCRIPTION OF THE DRAWING

Figure is a graph showing change of conversions in the preparation of CTFE according to the present invention.

DETAILED DESCRIPTION OF THE INVENTION

In the process of the present invention, as the catalyst, $Ni/SiO_2$, Ni/MgO, Ni/Zeolite Y; iron, tin, zinc and chromium as well as their oxides such as $Fe_2O_3$, $SnO_2$, ZnO and $Cr_2O_3$; nickel/oxide and copper oxide can be used.

The catalyst component selected from the group consisting of iron, tin, zinc, chromium and their oxides, nickel oxide and copper oxide can be carried on a support.

Thereby, the activity of the catalyst is improved so that the selectivity is increased, and a life of the catalyst is prolonged.

Preferred examples of the support are active carbon, $SiO_2$, MgO, $Al_2O_3$, $ZrO_2$, Zeolite Y, silica-alumina, silicon carbide, diatomaceous earth.

Preferred combinations of the catalyst and the support are $Ni/SiO_2$, $Ni/MgO$, $Ni/Zeolite$ Y, $Fe/SiO_2$, $Cr/SiO_2$.

The catalyst component may be carried on the support in an amount of from 0.1 to 50 % by weight based on the total weight.

The molar ratio of 1,1,2-trichloro-1,2,2-trifluoroethane to hydrogen is not critical. Preferably, 1 to 10 moles, more preferably 1 to 5 moles of hydrogen is used per one mole of 1,1,2-trichloro-1,2,2-trifluoroethane. As the excessive amount of hydrogen decreases, the selectivity decreases. When the excessive amount of hydrogen is too large, a ratio of utilized hydrogen decreases.

The reaction temperature is from 300 to 550°C, preferably from 400 to 500°C. At a temperature lower than 300°C, the conversion decreases, while at a temperature higher than 550°C, the selectivity decreases and/or the catalyst is deteriorated. The reaction pressure is not critical. The process of the present invention can be carried out under any practically employed pressure.

The space velocity (SV) of the raw materials is preferably from 100 to 20,000 $hr^{-1}$. SV is defined by a ratio of a volume of the raw materials supplied in a unit time at a reaction temperature to an apparent packed volume of the catalyst.

The gaseous reactants materials can be supplied with or without dilution with an inert gas such as nitrogen, argon or helium which has no influence on the reaction.

The process of the present invention is preferably carried out in the presence of steam, since steam is extremely effective for preventing deterioration of the catalyst. An amount of steam is up to 20 % by volume of all the volume of the charged materials.

PREFERRED EMBODIMENTS OF THE INVENTION

The present invention will be illustrated by following Examples.

Example 1

Each catalyst used in this Example was prepared by dispersing silica (Aerosil) or magnesium oxide in an aqueous solution of a nitrate of each metal, evaporating the dispersion to dryness, and sieving the residual solid to particle sizes of 32 mesh to 60 mesh.

The reaction was carried out in a fixed bed flow reactor under atmospheric pressure.

One gram of the catalyst was charged in the reactor and pre-treated in an argon stream at 450°C for 2 hours. Then, at the same temperature, the raw materials were supplied to start the reaction. The raw material mixture consisted of 1,1,2-trichloro-1,2,2-trifluoroethane, hydrogen and argon which were supplied at flow rates of 10 ml/min., 29 ml/min. and 56 ml/min., respectively. By-produced hydrogen chloride was removed by absorbing it with an aqueous solution of potassium hydroxide, and the produced materials were analyzed with gas chromatography.

Figure shows change of conversions with time when the catalyst comprising $NiO$ or $Fe_2O_3$ supported on the above carrier was used.

The used catalysts clearly showed the activity on the reaction of the present invention.

The activity of 20 wt.% $NiO/SiO_2$ was extremely high. During the 8 hour reaction, the conversion was 100 % and the selectivity was 80 %.

To study the characteristics of the supported $NiO$ catalyst more precisely, the reaction was carried out by reducing the supported amount of $NiO$ to 10 % by weight and the amount of the supported catalyst to 0.2 g (one fifth). The results are also shown in Figure. The activity slightly increased in an initial stage of the reaction. Though the amount of $NiO$ was only one tenth of the 20 wt.% $Fe_2O_3/SiO_2$, the conversion was about 70 % which was about six times larger than in case of the 20 wt.% $Fe_2O_3/SiO_2$. The selectivity was 100 %.

The X-ray diffraction analysis of the catalysts before and after reaction revealed that $NiO$ was reduced to metal nickel during the reaction. Actually, the $NiO$ catalyst which had been reduced with hydrogen before use had the same catalytic activity as the non-reduced $NiO$ catalyst.

Example 2

CTFE was prepared by using various supported catalysts. As the supported catalyst, $Ni/SiO_2$ (1, 2, 5, 10, 20 or 30 % by weight of Ni), $Ni/MgO$ (20 % by weight of Ni), Ni/active carbon, Ni/Zeolite Y, $Fe/SiO_2$ and $Cr/SiO_2$ were prepared. The preparation procedures were as follows:

(1) Ni/SiO$_2$

To silica (Aerosil) (20 g), 107 ml of an aqueous solution of nickel nitrate (Ni(NO$_3$)$_2$.5H$_2$O) in a concentration shown in Table 1 was dropwise added. After completing the addition of the aqueous solution, wet silica was well kneaded and then mixed in the air at 70°C for one day followed by sintering at 450°C for 6 hours. As silica, from very fine particle one such as Aerosil to coarse one such as quartz sand can be used.

Table 1

| % by weight | Gram |
|---|---|
| 1 | 0.79 |
| 2 | 1.59 |
| 5 | 4.10 |
| 10 | 8.65 |
| 20 | 19.47 |
| 30 | 33.37 |

(2) Ni/MgO

MgO (20 g) was dispersed in water (500 ml). To the dispersion, 200 ml of an aqueous solution of nickel nitrate (19.5 g) was dropwise added and the mixture was stirred at room temperature for one day. Then, the mixture was heated at 80 to 90°C to evaporate water off. The residue was further dried at 120°C for one day followed by sintering in the air at 600°C for 5 minutes.

(3) Ni/active carbon (Comparative Example)

To active carbon of 32 to 60 mesh (4.54 g), 8 ml of an aqueous solution of nickel nitrate (4.42 g) was dropwise added, and resulting wet active carbon was well kneaded. Then, the wet active carbon was dried at 120°C for one day. The dried product was used without sintering at high temperature.

(4) Ni/Zeolite Y

Zeolite Y (9 g) was added to 200 ml of a 0.2 N aqueous solution of nickel chloride, and kept at 70°C for 6 hours while stirring. After cooling no room temperature, the mixture was filtered, and the filter cake was dried at 120°C for one day. This product was used without sintering at high temperature as in case of the Ni/active carbon.

(5) Fe/SiO$_2$

By the same procedures as in the preparation of Ni/MgO but eliminating the one day stirring and using 25.3 g of iron nitrate (Fe(NO$_3$)$_3$.9H$_2$O) per 20 g of SiO$_2$, the Fe/SiO$_2$ catalyst was prepared.

(6) Cr/SiO$_2$

By the same procedures as in the preparation of Fe/SiO$_2$ but using 26.3 g of chromium nitrate (Cr-(NO$_3$)$_3$.9H$_2$O) per 20 g of SiO$_2$, the Cr/SiO$_2$ catalyst was prepared.

By using each of the above catalysts, CTFE was prepared by supplying 1,1,2-trichloro-1,2,2-trifluoroethane, hydrogen and argon at flow rates of 10 ml/min., 29 ml/min. and 56 ml/min., respectively under the conditions specified in Table 2. The results are also shown in Table 2.

Table 2

| Metal or oxide | Carrier[1] | Supported amount (wt.%) | Pre-reduction[2] with hydrogen | Amount of catalyst (g) | Space velocity ($hr^{-1}$) | Contact time (sec.) | Reaction temp. (°C) | Conversion (%) | Selectivity (%) | Yield (%) |
|---|---|---|---|---|---|---|---|---|---|---|
| Ni | $SiO_2$ | 1.0 | Yes | 0.2 | 11,400 | 0.3 | 450 | 23.6 | 100 | 29.0 |
|  |  | ↑ | ↑ | 1.0 | 2,280 | 1.6 | ↑ | 94.2 | 100 | 94.2 |
|  |  | 2.0 | ↑ | 0.2 | 11,400 | 0.3 | ↑ | 45.4 | 100 | 45.4 |
|  |  | ↑ | ↑ | 1.0 | 2,280 | 1.6 | ↑ | 100 | 95.8 | 95.8 |
|  |  | 10.0 | No | 0.2 | 11,400 | 0.3 | ↑ | 65.0 | 98.9 | 64.3 |
|  |  | ↑ | Yes | ↑ | ↑ | ↑ | ↑ | 100 | 91.2 | 91.2 |
|  |  | 30.0 | ↑ | ↑ | ↑ | ↑ | ↑ | 58.3 | 85.3 | 49.7 |
|  |  | ↑ | ↑ | ↑ | ↑ | ↑ | 440 | 43.2 | 77.0 | 33.0 |
|  |  | ↑ | ↑ | ↑ | ↑ | ↑ | 430 | 22.0 | 100 | 22.0 |
|  |  | ↑ | ↑ | ↑ | ↑ | ↑ | 420 | 13.0 | 100 | 13.0 |
|  |  | ↑ | ↑ | ↑ | ↑ | ↑ | 410 | 6.4 | 100 | 6.4 |
| Ni | MgO | 20.0 | Yes | 1.0 | 4,071 | 0.9 | 450 | 30.7 | 88.8 | 27.3 |
|  |  | ↑ | No | ↑ | ↑ | ↑ | ↑ | 92.6 | 96.8 | 89.6 |
| Ni[4] | Active C[4] | 20.0 | Yes | 1.0 | 3,563 | 1.0 | 450 | 39.5 | 67.1 | 26.5 |
| Ni | Zeolite Y | 18.0 | Yes | 1.0 | 2,478 | 1.5 | 450 | 96.0 | 94.8 | 91.0 |
| Fe | $SiO_2$ | 20.0 | No | 1.0 | 2,280 | 1.6 | 450 | 13.0 | 97.9 | 12.7 |
| Cr | $SiO_2$ | 20.0 | No | 1.0 | 2,280 | 1.6 | 450 | 39.2 | 0 | 0?? |
| NiO | -- | -- | No | 1.0 | 4,750 | 0.8 | 450 | 72.2 | 89.4 | 64.5 |
| NiO[3] | -- | -- | No | 0.5 | 10,000 | 0.4 | 450 | 88.7 | 94.7 | 84.0 |

Note: 1) Particle size: 32 to 60 mesh.
2) Pre-treatment with hydrogen at a flow rate of 29 ml/min. at 450°C for 2 hours.
3) Flow rates: 1,1,2-trichloro-1,2,2-trifluoroethane, 7.2 ml/min.; $H_2$, 2.5 ml/min.; Ar, 70 ml/min.
4) Comparative Example

Example 3

By using a catalyst consisting of 0.5 g of nickel oxide and 3.0 g of quartz sand and supplying 1,1,2-trichloro-1,2,2-trifluoroethane, hydrogen, argon and steam at flow rates 7.2 ml/min., 20 ml/min., 70 ml/min.

and 2.5 ml/min., respectively at 450°C, CTFE was prepared. After 24 hours, the conversion was maintained at 95 %, and the selectivity was maintained at 90 %.

When no steam was supplied, after 9 hours, the conversion decreased to 84 % and the selectivity decreased to 83 %.

Example 4

Commercially available $Fe_2O_3$ (reagent grade) was sieved to 32 to 60 mesh and one gram of $Fe_2O_3$ was filled in a reactor tube. After pre-treating $Fe_2O_3$ in an argon stream at 450°C for 2 hours, the raw material mixture was supplied at following flow rates at the same temperature:

1,1,2-trichloro-1,2,2-trifluoroethane: 10 ml/min.

Hydrogen: 29 ml/min.

Argon: 56 ml/min.

The resulting reaction mixture was washed with water, dried and analyzed by gas chromatography. The conversion of 1,1,2-trichloro-1,2,2-trifluoroethane was 28 % and the selectivity of CTFE was 98.9 %.

Examples 5 - 8

In the same manner as in Example 4 but using the catalyst of Table 3, the reaction was carried out and the resulting reaction mixture was analyzed. The results are shown in Table 3.

Table 3

| Example No | Catalyst | Conversion (%) | Selectivity (%) |
|---|---|---|---|
| 5 | $SnO_2$ | 9.1 | 76.5 |
| 6 | NiO | 56.1 | 88.7 |
| 7 | CuO | 10.3 | 73.4 |
| 8 | ZnO | 4.2 | 73.5 |

**Claims**

1. A process for preparing chlorotrifluoroethylene which comprises reacting 1,1,2-trichloro-1,2,2-trifluoroethane with hydrogen in the presence of a catalyst selected from the group consisting of $Ni/SiO_2$, Ni/MgO, Ni/Zeolite Y, iron, tin, zinc, chromium and their oxides, nickel oxide and copper oxide.

2. The process according to Claim 1, wherein the catalyst selected from the group consisting of iron, tin, zinc, chromium and their oxides, nickel oxide and copper oxide is carried on a carrier.

3. The process according to Claim 2, wherein said carrier is selected from the group consisting of active carbon, $SiO_2$, MgO, $Al_2O_3$, $ZrO_2$, Zeolite Y, silica-alumina, silicon carbide and diatomaeous earth.

4. The process according to Claim 2, wherein said catalyst is selected from the group consisting of $Fe/SiO_2$ and $Cr/SiO_2$.

5. The process according to any of the preceding claims, wherein the molar ratio of hydrogen to 1,1,2-trichloro-1,2,2-trifluoroethane is from 1:1 to 10:1.

6. The process according to any of the preceding claims, wherein the molar ratio of hydrogen to 1,1,2-trichloro-1,2,2-trifluoroethane is from 1:1 to 5:1.

7. The process according to any of the preceding claims, wherein the reaction temperature is from 300 to 550 °C.

8. The process according to any of the preceding claims, wherein the reaction is carried out at a space velocity of from 100 to 20000 h$^{-1}$.

9. The process according to any of the preceding claims, wherein the reaction is carried out in the presence of steam.

**Patentansprüche**

1. Verfahren zur Herstellung von Chlortrifluorethylen, umfassend die Reaktion von 1,1,2-Trichlor-1,2,2-trifluorethan mit Wasserstoff in Gegenwart eines Katalysators, ausgewählt aus der Gruppe bestehend aus Ni/SiO$_2$, Ni/MgO, Ni/Zeolit Y, Eisen, Zinn, Zink, Chrom und deren Oxiden, Nickeloxid und Kupferoxid.

2. Verfahren gemäss Anspruch 1, dadurch **gekennzeichnet,** dass der aus der Gruppe, bestehend aus Eisen, Zinn, Zink, Chrom und deren Oxiden, Nickeloxid und Kupferoxid, ausgewählte Katalysator auf einen Träger aufgebracht ist.

3. Verfahren gemäss Anspruch 2, dadurch **gekennzeichnet,** dass der Träger ausgewählt ist aus der Gruppe bestehend aus Aktivkohle, SiO$_2$, MgO, Al$_2$O$_3$, ZrO$_2$, Zeolit Y, Silicium-Aluminiumoxid, Siliciumcarbid und Diatomeenerde.

4. Verfahren gemäss Anspruch 2, dadurch **gekennzeichnet,** dass der Katalysator ausgewählt ist aus der Gruppe bestehend aus Fe/SiO$_2$ und Cr/SiO$_2$.

5. Verfahren gemäss mindestens einem der vorhergehenden Ansprüche, dadurch **gekennzeichnet,** dass das Molverhältnis von Wasserstoff zu 1,1,2-Trichlor-1,2,2-trifluorethan im Bereich von 1:1 bis 10:1 liegt.

6. Verfahren gemäss mindestens einem der vorhergehenden Ansprüche, dadurch **gekennzeichnet,** dass das Molverhältnis von Wasserstoff zu 1,1,2-Trichlor-1,2,2-trifluorethan im Bereich von 1:1 bis 5:1 liegt.

7. Verfahren gemäss mindestens einem der vorhergehenden Ansprüche, dadurch **gekennzeichnet,** dass die Reaktionstemperatur im Bereich von 300 bis 550°C liegt.

8. Verfahren gemäss mindestens einem der vorhergehenden Ansprüche, dadurch **gekennzeichnet,** dass die Reaktion durchgeführt wird mit einer Raumgeschwindigkeit von 100 bis 20.000 h$^{-1}$.

9. Verfahren gemäss mindestens einem der vorangehenden Ansprüche, dadurch **gekennzeichnet,** dass die Reaktion in Gegenwart von Dampf durchgeführt wird.

**Revendications**

1. Procédé de préparation de chlorotrifluoroéthylène qui comprend la réaction de 1,1,2-trichloro-1,2,2-trifluoroéthane avec de l'hydrogène en présence d'un catalyseur choisi dans le groupe constitué du Ni/SiO$_2$, du Ni/MgO, du Ni/zéolite Y, du fer, de l'étain, du zinc, du chrome et de leurs oxydes, de l'oxyde de nickel et de l'oxyde de cuivre.

2. Procédé selon la revendication 1, dans lequel le catalyseur choisi dans le groupe constitué du fer, de l'étain, du zinc, du chrome et de leurs oxydes, de l'oxyde de nickel et de l'oxyde de cuivre est porté sur un support.

3. Procédé selon la revendication 2, dans lequel on choisit ledit support dans le groupe constitué du charbon actif, du SiO$_2$, du MgO, de l'Al$_2$O$_3$, du ZrO$_2$, de la zéolite Y, de la silice-alumine, du carbure de silicium et de la diatomite.

4. Procédé selon la revendication 2, dans lequel on choisit ledit catalyseur dans le groupe constitué du Fe/SiO$_2$ et du Cr/SiO$_2$.

7

5. Procédé selon l'une quelconque des revendications précédentes, dans lequel le rapport molaire de l'hydrogène au 1,1,2-trichloro-1,2,2-trifluoroéthane est compris entre 1:1 et 10:1.

6. Procédé selon l'une quelconque des revendications précédentes, dans lequel le rapport molaire de l'hydrogène au 1,1,2-trichloro-1,2,2-trifluoroéthane est compris entre 1:1 et 5:1.

7. Procédé selon l'une quelconque des revendications précédentes, dans lequel la température de réaction est comprise entre 300 et 550 °C.

8. Procédé selon l'une quelconque des revendications précédentes, dans lequel on met en oeuvre la réaction à une vitesse spatiale comprise entre 100 et 20 000 h$^{-1}$.

9. Procédé selon l'une quelconque des revendications précédentes, dans lequel on met en oeuvre la réaction en présence de vapeur.

Figure